# EUROPÄISCHE PATENTSCHRIFT

(11) **EP 0 864 324 B1**
(45) Veröffentlichungstag und Bekanntmachung des Hinweises auf die Patenterteilung: **15.10.2003**
(21) Anmeldenummer: 98104493.6
(22) Anmeldetag: 12.03.1998
(51) Int. Cl.: A61K 9/20

(54) **Verfahren zur Herstellung von festen Kombinationsarzneiformen**
Method to produce solid combined pharmaceutical forms
Méthode pour la production de formes pharmaceutiques solides combinées

(30) Priorität: 12.03.1997 DE 19710213
(43) Veröffentlichungstag der Anmeldung: 16.09.1998
(73) Patentinhaber: BASF AKTIENGESELLSCHAFT, 67056 Ludwigshafen (DE)
(72) Erfinder: Zeidler, Jürgen, 67122 Mutterstadt (DE); Rosenberg, Joerg, 67158 Ellerstadt (DE); Breitenbach, Jörg, 68199 Mannheim (DE); Kleinke, Andreas, 67063 Ludwigshafen (DE); Maier, Werner, 67105 Schifferstadt (DE)
(74) Vertreter: Kinzebach, Werner, Dr.

(56) Entgegenhaltungen:
- WO-A-97/15293
- DE-A- 4 446 468
- FR-A- 2 073 278

## Beschreibung

Die vorliegende Erfindung betrifft ein Verfahren zur Herstellung von festen, mindestens zweiphasigen Kombinationsarzneiformen auf Basis von mindestens einem pharmakologisch akzeptablen polymeren Bindemittel und mindestens einem pharmazeutischen Wirkstoff sowie die dadurch erhältlichen Kombinationsarzneiformen.

Die klassischen Verfahren zur Herstellung fester Arzneiformen, insbesondere Tabletten, werden diskontinuierlich durchgeführt und umfassen mehrere Stufen. Im allgemeinen werden hierfür pharmazeutische Granulate als Ausgangsmaterial verwendet. Sowohl die Herstellung der Granulate als auch der festen Arzneiformen ist zeit- und kostenintensiv. Dies gilt insbesondere dann, wenn Kombinationspräparate hergestellt werden sollen, um unterschiedliche Wirkstoffe in einer Arzneiform aufzunehmen oder um unterschiedliche Freisetzungscharakteristiken zu erzielen.

Seit einiger Zeit ist ein wesentlich einfacheres kontinuierliches Verfahren zur Herstellung fester Arzneiformen bekannt, bei dem man eine wirkstoffhaltige lösungsmittelfreie Schmelze aus einem polymeren wirkstoffhaltigen Bindemittel extrudiert und den extrudierten Strang zu der gewünschten Arzneiform formt, beispielsweise in einem Kalander mit Formwalzen, siehe EP-A-240 904, EP-A-240 906, EP-A-337 256 und EP-A-358 105. Dieses Verfahren erlaubt es jedoch nicht, zwei miteinander nicht verträgliche Wirkstoffe in einer Arzneiform zu kombinieren oder unterschiedliche Freisetzungscharakteristiken zu verwirklichen.

Die DE-A-44 46 468.4 beschreibt die Herstellung von umhüllten Tabletten aus einer wirkstoffhaltigen Polymerschmelze mit Hilfe eines Kalanders mit Formwalzen, wobei man die wirkstoffhaltige Polymerschmelze zwischen zwei Folien aus dem Umhüllungsmaterial in die Formwalzen einführt. Dieses Verfahren ermöglicht es zwar, einen weiteren Wirkstoff in den Folien vorzusehen und die Tabletten beispielsweise mit einem Magensaft-resistenten Überzug zu versehen. Hinsichtlich der Art und Menge der Wirkstoffe in den Folien sind aber enge Grenzen gesetzt, so daß die auf diese Weise hergestellten Tabletten nicht universell anwendbar sind.

Die WO-A-9715293 (relevant gemäß Art 54(6) EPO) (nicht vorveröffentlichte) DE 195 39 361.9 beschreibt die Koextrusion von mindestens zwei Polymerschmelzen, wobei mindestens eine wirkstoffhaltig ist und wobei man, je nach Koextrusionswerkzeug, Tabletten aus mindestens zwei übereinanderliegenden Schichten oder aus mindestens zwei koaxial angeordneten Schichten (Kern-Hülle) erhält.

FR-A- 2073278 offenbart zweiphasige kombinationsarzneiformen, bei der als zweite Phase ein Kern so in die erste Phase eingelagert ist, daß ein Teil des Kerns sichtbar bleibt. Die Präparate werden durch Kompression erhalten.

Der vorliegenden Erfindung liegt die Aufgabe zugrunde, ein Verfahren zur Herstellung von festen Kombinationsarzneiformen zur Verfügung zu stellen, das einfach und allgemein anwendbar ist und die Herstellung derartiger Kombinationsarzneiformen in großer Variationsbreite ermöglicht.

Überraschenderweise wurde nun gefunden, daß diese Aufgabe gelöst wird, wenn man beim Ausformen einer Arzneiform aus einer Schmelze aus einem polymeren Bindemittel mindestens ein festes Präparat in die noch plastische Masse aufnimmt, wobei die Schmelze und/oder das Präparat wenigstens einen Wirkstoff enthalten.

Gegenstand der vorliegenden Erfindung ist daher ein Verfahren zur Herstellung von festen, mindestens zweiphasigen Kombinationsarzneiformen auf Basis von mindestens einem pharmakologisch akzeptablen polymeren Bindemittel und mindestens einem pharmazeutischen Wirkstoff, das dadurch gekennzeichnet ist, daß man eine Schmelze aus dem polymeren Bindemittel (Bindemittel A) und dem gegebenenfalls mindestens einem Wirkstoff ausformt, wobei beim Ausformen mindestens ein festes Präparat, das gegebenenfalls mindestens einen Wirkstoff enthält, in die noch plastische Masse aufgenommen wird.

Für die Herstellung der Schmelze ist es erforderlich, die Bestandteile, nämlich mindestens ein pharmakologisch akzeptables polymeres Bindemittel (Bindemittel A), gegebenenfalls mindestens einen pharmazeutischen Wirkstoff und gegebenenfalls übliche Additive, zu vermischen und zu einem plastischen Gemisch aufzuschmelzen, vorzugsweise in Abwesenheit eines Lösungsmittels. Diese Verfahrensschritte können auf bekannte Art und Weise durchgeführt werden, beispielsweise wie in der EP-A-240 904, EP-A-337 256 und EP-A-358 105 beschrieben ist.

Die Komponenten können zuerst vermischt und dann aufgeschmolzen und homogenisiert werden. Insbesondere bei Verwendung von empfindlichen Wirkstoffen hat es sich aber als bevorzugt erwiesen, zuerst das polymere Bindemittel, gegebenenfalls zusammen mit üblichen pharmazeutischen Additiven, aufzuschmelzen und vorzuvermischen, wobei die Apparaturen, wie Rührkessel, Rührwerke, Feststoffmischer etc., gegebenenfalls im Wechsel betrieben werden, und dann den (die) empfindlichen Wirkstoff(e) in "Intensivmischern" in plastischer Phase bei sehr kleinen Verweilzeiten einzumischen (Homogenisieren). Der (die) Wirkstoff(e) kann (können) in fester Form oder als Lösung oder Dispersion eingesetzt werden.

Das Aufschmelzen und Vermischen erfolgt in einer für diesen Zweck üblichen Vorrichtung. Besonders geeignet sind Extruder oder beheizbare Behälter mit Rührwerk, z.B. Kneter, (wie der unten noch erwähnten Art).

Als Mischapparat sind auch solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z.B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z.B. Dispax der Firma IKA).

Bei empfindlichen Wirkstoffen erfolgt vorzugsweise zunächst das Aufschmelzen des polymeren Bindemittels in einem Extruder und anschließend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Das Beschicken der Mischvorrichtung erfolgt je nach deren Konzeption kontinuierlich oder diskontinuierlich in üblicher Weise. Pulverförmige Komponenten können im freien Zulauf, z.B. über eine Differentialdosierwaage eingeführt werden. Plastische Massen können direkt aus einem Extruder eingespeist oder über eine Zahnradpumpe, die insbesondere bei hohen Viskositäten und hohen Drücken von Vorteil ist, zugespeist werden. Flüssige Medien können über ein geeignetes Pumpenaggregat zudosiert werden.

Das durch Vermischen und Aufschmelzen des Bindemittels, gegebenenfalls des Wirkstoffes und gegebenenfalls des Additivs oder der Additive erhaltene Gemisch ist teigig bis zähflüssig (thermoplastisch) und daher auch extrudierbar. Das Bindemittel soll vorzugsweise in physiologischer Umgebung löslich oder quellbar sein. Beispiele für geeignete Bindemittel sind:
Polyvinylpyrrolidon (PVP), Copolymerisate von N-Vinylpyrrolidon (NVP) und Vinylestern, insbesondere Vinylacetat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate (Eudragit-Typen), Copolymerisate von Methylmethacrylat und Acrylsäure, Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane. Die K-Werte (nach H. Fikentscher, Cellulose-Chemie 13 (1932), Seiten 58 bis 64 und 71 und 74) der Polymere liegen im Bereich von 10 bis 100, vorzugsweise 12 bis 70, insbesondere 12 bis 35, für PVP > 17, insbesondere 20 bis 35.

Brauchbar sind auch bioabbaubare Polymere, wie Polyhydroxyalkanoate, z.B. Polyhydroxybuttersäure, Polymilchsäure, Polyaminosäuren, z.B. Polylysin, Polyasparagin, Polydioxane u. Polypeptide.

Bevorzugte polymere Bindemittel sind Polyvinylpyrrolidon, Copolymerisate von N-Vinylpyrrolidon und Vinylestern, Polyhydroxyalkylacrylate, Polyhydroxyalkylmethacrylate, Polyacrylate, Polymethacrylate, Alkylcellulosen und Hydroxyalkylcellulosen.

Das polymere Bindemittel muß in der Gesamtmischung aller Komponenten im Bereich von 50 bis 180°C, vorzugsweise 60 bis 130°C erweichen oder schmelzen. Die Glasübergangstemperatur der Mischung muß daher unter 180°C, vorzugsweise unter 130°C liegen. Erforderlichenfalls wird sie durch übliche, pharmakologisch akzeptable weichmachende Hilfsstoffe herabgesetzt. Die Menge an Weichmacher beträgt höchstens 30 Gew.-%, bezogen auf das Gesamtgewicht von Bindemittel und Weichmacher, damit lagerstabile Arzneiformen gebildet werden, die keinen kalten Fluß zeigen. Vorzugsweise aber enthält das Gemisch keinen Weichmacher.

Beispiele für derartige Weichmacher sind:
langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylen-propylenglykole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im allgemeinen 0,5 bis 15, vorzugsweise 0,5 bis 5 Gew.-%, bezogen auf das Gesamtgewicht des Gemisches.

Übliche galenische Hilfsstoffe, deren Gesamtmenge bis zu 100 Gew.-% bezogen auf das Polymerisat, betragen kann, sind z.B. Streckmittel bzw. Füllstoffe, wie Silikate oder Kieselerde,
Magnesiumoxid, Aluminiumoxid, Titanoxid, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches;
Schmiermittel und Trennmittel wie Magnesium-, Aluminium- und Calciumstearat, Talcum und Silicone, sowie tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Schmier- und Trennmitteln beträgt vorzugsweise 0,1 bis 5 Gew.-%, bezogen auf das Gesamtgewicht der Masse für die jeweilige Schicht;
Fließmittel, z.B, Aerosil, in einer Menge von 0,1 bis 5 Gew.-%, bezogen auf das Gesamtgewicht des Gemisches;
Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;
Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn- und Treibmittel zugesetzt werden (vgl. z.B. H. Sucker et al. Pharmazeutische Technologie, Thieme-Verlag, Stuttgart 1978).

Unter Hilfsstoffen im Sinne der Erfindung sind auch Substanzen zur Herstellung einer festen Lösung mit dem pharmazeutischen Wirkstoff zu verstehen. Diese Hilfsstoffe sind beispielsweise Pentaerythrit und Pentaerythrit-tetracaetat, Polymere wie z.B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z.B. bei J. L. Ford, Pharm. Acta Helv. 61, 69-88 (1986) an-5 gegeben.

Als pharmazeutische Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Einzige Voraussetzung für die Eignung von Hilfsstoffen ist eine ausreichende Temperaturstabilität.

Das Ausformen des plastischen Gemisches erfolgt ebenfalls in üb-5 licher Weise. Vorzugsweise erfolgt das Ausformen mit Hilfe eines Kalanders mit zwei Formwalzen, die sich entlang einer Mantellinie berühren und gegenläufig rotieren. Die Formwalzen weisen auf ihrer Oberfläche Vertiefungen auf, die der Form einer Hälfte der gewünschten Arzneiform entsprechen. Im Berührungsbereich der beiden Walzen erfolgt die Formgebung der Arzneiform durch Kombination der Tablettenmasse einer Vertiefung auf der einen Walze mit derjenigen der gegenüberliegenden Vertiefung auf der anderen Walze.

Die Form der Vertiefungen und damit der Arzneiform kann weitgehend beliebig gewählt werden. Besonders zweckmäßig sind längliche und ellipsoid segmentartige Vertiefungen, so daß man Oblongtabletten oder linsenförmige Tabletten erhält. Dabei können die Vertiefungen auf der einen Formwalze von den Vertiefungen auf der anderen Formwalze verschieden sein. Außerdem kann eine Formwalze mit Vertiefungen mit einer Glattwalze kombiniert werden. Weiter hat es sich in manchen Fällen als zweckmäßig erwiesen, die Vertiefungen mit einem Formentrennmittel zu überziehen, um das Ablösen der Arzneiform von der Formwalzen zu erleichtern. Geeignete Formentrennmittel sind beispielsweise Silikonharze, Stearinsäure, Calcium- oder Magnesiumstearat, Paraffin, Cetylalkohol oder Lecithine.

Die Formwalzen können heiz- oder kühlbar sein, vorzugsweise wird zumindest diejenige Formwalze gekühlt, in deren Vertiefungen die wirkstoffhaltige Schmelze aus dem Bindemittel (B) gegeben wird (siehe unten).

Die Schmelze wird den Formwalzen in üblicher Weise zugeführt, vorzugsweise jedoch als extrudierter Strang oder extrudiertes Band.

Beim Ausformen wird in die noch plastische Masse mindestens ein festes Präparat aufgenommen, das gegebenenfalls mindestens einen Wirkstoff enthält. Wenn die Schmelze aus dem polymeren Bindemittel einen Wirkstoff enthält, kann es sich um den gleichen oder einen anderen Wirkstoff handeln. Man erhält auf diese Weise (nach dem Abkühlen) eine feste Kombinationsarzneiform aus mindestens zwei Phasen, wobei die erste Phase das polymere Bindemittel (A), gegebenenfalls mindestens einen pharmazeutischen Wirkstoff und gegebenenfalls pharmazeutische Additive umfaßt. Die aus dem festen Präparat gebildete zweite Phase kann so in die erste Phase eingelagert sein, daß ein Teil ihrer Oberfläche sichtbar bleibt (d.h. die Arzneiform weist keine Kern-Mantel-Struktur auf). Sie kann jedoch auch vollständig in die erste Phase aufgenommen werden.

Als festes Präparat ist jede feste Arzneiform geeignet, vorausgesetzt sie "verschmilzt" nicht homogen mit der ersten Phase, sondern ist in der Lage, eine eigene Phase zu bilden. Geeignete feste Präparate sind beispielsweise Tabletten, eine erstarrte wirkstoffhaltige Schmelze aus einem pharmakologisch akzeptablen, polymeren Bindemittel (Bindemittel B), eine erstarrte Wirkstoffschmelze, Pillen, Pellets oder Wirkstoffkristalle, die den Wirkstoff in einem üblichen Träger, wie Alkylcellulosen, beispielsweise Methyl- oder Ethylcellulose, Hydroxyalkylcellulosen, beispielsweise Hydroxyethyl- oder Hydroxypropylcellulose, Stärke, Milchsäure, Zucker, wie Milchzucker, Sorbit, Isomalt, Calciumhydrogenphosphat etc., enthalten. Das feste Präparat kann unbeschichtet oder in üblicher Weise beschichtet sein, z.B. mit den unten noch genannten Filmüberzügen.

Vorzugsweise verwendet man als festes Präparat konventionelle Tabletten oder Filmtabletten.

Besonders bevorzugt ist es, die wirkstoffhaltige Schmelze aus dem polymeren Bindemittel (B) oder die Wirkstoffschmelze in den Vertiefungen einer Formwalze erstarren zu lassen. Zu diesem Zweck wird die Schmelze in der gewünschten Menge und in niedrig-viskoser Form in die Vertiefungen von mindestens einer Formwalze gegeben. Dort läßt man sie erstarren, beispielsweise durch Aufblasen von gekühlter Luft oder durch Kühlung der Formwalze. Die Teile aus der erstarrten Schmelze werden dann beim Ausformen in die noch plastische Schmelze aus dem wirkstoffhaltigen polymeren Bindemittel (A) aufgenommen.

Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht wesentlich zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkonzentration im Bereich von 0,001 bis 95, vorzugsweise von 20 bis 80, insbesondere 30 bis 70 Gew.-% liegen. Auch Wirkstoff-Kombinationen können eingesetzt werden. Wirkstoffe im Sinne der Erfindung sind auch Vitamine und Mineralstoffe, sowie Pflanzenbehandlungsmittel und Insektizide. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z.B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I-und J-Gruppe, K-Gruppe und P-Gruppe. Zu Wirkstoffen im Sinne der Erfindung gehören auch Peptidtherapeutika und Impfstoffe.

Das erfindungsgemäße Verfahren ist beispielsweise zur Verarbeitung folgender Wirkstoffe bzw. der pharmakologisch aktiven Salze davon geeignet:
Acebutolol, Acetylcystein, Acetylsalicylsäure, Acyclovir, Albrazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefatroxil, Cefazolin, Cefixim, Cefotaxim, Ceftazidim, Ceftriaxon, Cefuroxim, Celedilin, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomibramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrat, Isotretinoin, Ketotifen, Ketoconazol, Ketoprofen, Ketorolac, Labatalon, Lactulose, Lecithin, Levocarnitin, Levodopa, Levoglutamid, Levonorgestrel, Levothyroxin, Lidocain, Lipase, Lipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Phenoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Promocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Volinsäure, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin, Captopril, Omeprazol, Ranitidin, Tramadol, Cyclosporin, Trandolapril und Peptidtherapeutika.

Im einzelnen kann es zur Ausbildung von festen Lösungen kommen. Der Begriff "feste Lösungen" ist dem Fachmann geläufig, beispielsweise aus der eingangs zitierten Literatur. In festen Lösungen von pharmazeutischen Wirkstoffen in Polymeren liegt der Wirkstoff molekulardispers im Polymer vor.

Mit dem erfindungsgemäßen Verfahren herstellbare feste, mindestens zweiphasige Kombinationsarzneiformen sind insbesondere Tabletten, vorzugsweise Oblongtabletten, Dragees, Pastillen und Pellets. Die erhaltenen Arzneiformen können abschließend auch in üblicher Weise mit Filmüberzügen versehen werden, welche die Wirkstofffreisetzung kontrollieren oder den Geschmack abdecken. Geeignete Materialien für derartige Überzüge sind Polyacrylate, wie die Eudragit-Typen, Celluloseester, wie die Hydroxypropylmethylcellulosephthalate, sowie Celluloseether, wie Ethylcellulose, Hydroxypropylmethylcellulose oder Hydroxypropylcellulose.

Das erfindungsgemäße Verfahren ermöglicht die Herstellung von Kombinationsarzneiformen, die einen oder mehrere Wirkstoffe enthalten können und zwar entweder in der erstarrten Schmelze aus dem Bindemittel (A) oder in dem festen Präparat oder in beiden. Dabei kann die Freisetzungscharakteristik des Wirkstoffes oder der Wirkstoffe durch Wahl entsprechender Bindemittel in gewünschter Weise variiert werden. Außerdem können nicht-kompatible Wirkstoffe miteinander kombiniert werden. Beispielsweise ist es möglich, Retardtabletten (SR = sustained release-Tabletten) mit einem IR-Anteil (IR = instant release) herzustellen. Diese sind vorteilhafterweise so aufgebaut, daß das feste Präparat den IR-Anteil bildet. Besonders geeignete Bindemittel hierfür sind Polyvinylpyrrolidone und Copolymerisate von N-Vinylpyrrolidon und Vinylacetat, sowie Zucker wie Milchzucker, Sorbit oder Isomalt. Der sustained release-Anteil enthält als Bindemittel insbesondere die oben genannten Cellulosederivate. Außerdem ist es möglich, SR/SRoder IR/IR-Kombinationen herzustellen. Entsprechende Überlegungen gelten für Kombinationsarzneiformen mit zwei oder mehreren verschiedenen Wirkstoffen.

Ein weiterer Gegenstand der vorliegenden Erfindung ist eine feste, mindestens zweiphasige Kombinationsarzneiform, die eine erste Phase aus mindestens einem pharmakologisch akzeptablen polymeren Bindemittel (Bindemittel A) in Form einer erstarrten Schmelze enthält, die gegebenenfalls mindestens einen pharmazeutischen Wirkstoff umfaßt und in die als zweite Phase ein festes Präparat, das mindestens einen Wirkstoff enthält, aufgenommen ist, wobei die zweite Phase so in die erste Phase eingelagert ist, dass ein Teil ihrer Oberfläche sichtbar bleibt.

Die nachfolgenden Beispiele erläutern die Erfindung ohne sie einzuschränken.

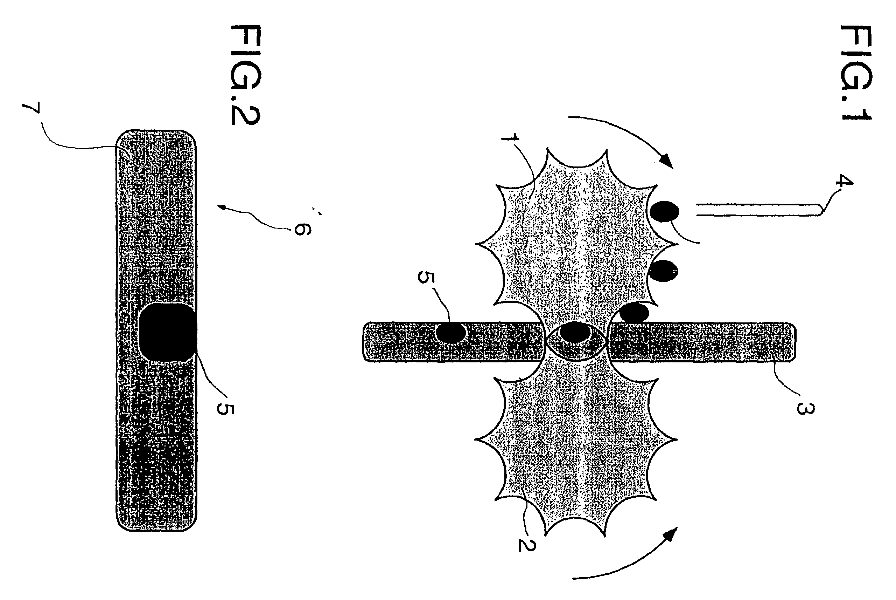

Die Herstellung einer erfindungsgemäßen Kombinationsarzneiform wird nachfolgend anhand der Figuren 1 und 2 näher erläutert. Es zeigen:
- Figur 1: eine schematische Darstellung des Herstellungsprozesses mit Hilfe von zwei Formwalzen
- Figur 2: einen Längsschnitt durch eine erfindungsgemäße Kombinationstablette, die nach der in Figur 1 gezeigten Methode erhalten wurde

In Figur 1 ist schematisch der Ausformvorgang mit Hilfe von zwei Formwalzen 1, 2 erläutert. Die Formwalzen 1, 2 drehen sich gegenläufig, die Drehrichtung ist durch die Pfeile angedeutet. Ein aus einem Extruder kommender Strang 3, der aus einem wirkstoffhaltigen polymeren Bindemittel besteht, wird in Pfeilrichtung zwischen die beiden Formwalzen 1, 2 eingeführt. Der Strang 3 liegt in plastischem Zustand vor. Gleichzeitig wird aus einer Zudosierstation 4 die gewünschte Menge einer Schmelze aus einem wirkstoffhaltigen polymeren Bindemittel (B) in die Vertiefungen der Formwalze 1 eingebracht. Die Formwalze 1 ist gekühlt, so daß die plastische Schmelze sofort erstarrt. Durch die gegenläufige Rotation der Formwalzen 1, 2 werden aus dem Strang 3 Tabletten 7 ausgestanzt, wobei in jede Tablette die in der jeweiligen Vertiefung befindliche erstarrte Schmelze 5 eingearbeitet wird. Falls erforderlich, werden die Tabletten abschließend gekühlt und gewünschtenfalls mit einem Filmüberzug versehen.

### Beispiel 1

Herstellung einer sustained release-Tablette mit instant release-Anteil:
Klucel EF (Hydroxypropylcellulose), Methocel K4M (Methylcellulose und Methylcellulosederivate, welche gleichzeitig Ethyl-, Hydroxyethyl-, Hydroxypropyl- oder Carboxymethylethergruppen enthalten), Lecithin und Verapamil-Hydrochlorid werden in einem Extruder in solchen Mengen vermischt und aufgeschmolzen, daß man eine Schmelze folgender Zusammensetzung erhält:

| | |
|---|---|
| Verapamil-Hydrochlorid | 48% |
| Klucel EF | 31,5% |
| Methocel K4M | 17,5% |
| Lecithin | 3% |

Vermischen, Aufschmelzen und Extrudieren erfolgen in einem Extruder unter folgenden Bedingungen:

| | Schuß 1 | Schuß 2 | Schuß 3 | Schuß 4 | Kopf |
|---|---|---|---|---|---|
| Temp. | 80°C | 100°C | 110°C | 110°C | 115°C |

Für den IR-Anteil wird Verapamil-Hydrochlorid in Isomalt oder Kollidon (Polyvinylpyrrolidon) oder einer Mischung davon gelöst bzw. dispergiert.

Diese Lösung bzw. Dispersion wird aus einer Zudosierstation in einer Menge von 50 mg in die Vertiefungen einer Formwalze eingebracht, vgl. die obige Figurenbeschreibung. Gleichzeitig wird der aus dem Extruder austretende Strang für den SR-Anteil mit Hilfe der Formwalzen wie oben beschrieben zu Oblongtabletten der in Figur 2 gezeigten Art ausgeformt.

### Beispiel 2

Man wiederholt das in Beispiel 1 angegebene Verfahren, wobei als IR-Anteil jedoch eine konventionelle IR-Tablette folgender Zusammensetzung verwendet wird:

| | |
|---|---|
| Verapamil-Ha | 40% |
| Ludipress¹⁾ | 59% |
| Magnesiumstearat | 1% |

| | |
|---|---|
| 1) Lactose-Monohydrat 43% Kollidon CC 3,5% Kollidon 30 3,5% | |

Eine entsprechende Tablette wird erhalten, wenn man als IR-Anteil Pellets folgender Zusammensetzung einsetzt:

| | |
|---|---|
| Paracetamol | 72% |
| Isomalt | 17,25% |
| Kollidon K30 | 4% |
| SDS | |
| (Natriumlaurylsulfat) | 0,75% |
| Prinojel | |
| (Natriumcarboxymethylstärke) | 5% |
| Hydriertes Rizinusöl | 1% |

## Patentansprüche

1. Verfahren zur Herstellung von festen, mindestens zweiphasigen Kombinationsarzneiformen auf Basis von mindestens einem pharmakologisch akzeptablen polymeren Bindemittel und mindestens einem pharmazeutischen Wirkstoff, **dadurch gekennzeichnet, daß** man eine Schmelze aus dem polymeren Bindemittel (Bindemittel A) und gegebenenfalls mindestens einem Wirkstoff ausformt, wobei beim Ausformen mindestens ein festes Präparat, das gegebenenfalls mindestens einen Wirkstoff enthält, in die noch plastische Masse aufgenommen wird.

2. Verfahren nach Anspruch 1, **dadurch gekennzeichnet, daß** man als festes Präparat eine erstarrte wirkstoffhaltige Schmelze aus einem pharmakologisch akzeptablen polymeren Bindemittel (Bindemittel B), eine erstarrte Wirkstoffschmelze, eine Tablette, Pille, ein Pellet oder Wirkstoffkristalle verwendet.

3. Verfahren nach Anspruch 2, **dadurch gekennzeichnet, daß** man als Bindemittel B eine Alkylcellulose, Hydroxyalkylcellulose, Carboxyalkylcellulose, Polyvinylpyrrolidon, ein Copolymerisat aus N-Vinylpyrrolidon und Vinylacetat, einen Zukkeralkohol oder ein Polyethylenglykol verwendet.

4. Verfahren nach Anspruch 1, **dadurch gekennzeichnet, daß** das Ausformen der Schmelze durch Extrudieren und Formgeben in einem Kalander mit gegenläufig rotierenden Formwalzen erfolgt, die auf ihrer Oberfläche Vertiefungen zur Aufnahme und Formung der Schmelze aufweisen.

5. Verfahren nach Anspruch 4, **dadurch gekennzeichnet, daß** man das feste Präparat in die Vertiefungen der einen Formwalze einbringt.

6. Verfahren nach Anspruch 5, **dadurch gekennzeichnet, daß** man die wirkstoffhaltige Schmelze aus dem polymeren Bindemittel B oder die Wirkstoffschmelze in die Vertiefungen der einen Formwalze einbringt und die Schmelze zum Erstarren bringt.

7. Verfahren nach einem der Ansprüche 4 bie 6, **dadurch gekennzeichnet, daß** man eine Formwalze mit Vertiefungen mit einer Glattwalze kombiniert.

8. Feste, mindestens zweiphasige Kombinationsarzneiform, mit einer ersten Phase aus mindestens einem pharmakologisch akzeptablen polymeren Bindemittel (Bindemittel A) in Form einer erstarrten Schmelze, die gegebenenfalls mindestens einen pharmazeutischen Wirkstoff umfaßt, in die als zweite Phase ein festes Präparat, das mindestens einen Wirkstoff enthält, aufgenommen ist, wobei die zweite Phase so in die erste Phase eingelagert ist, dass ein Teil ihrer Oberfläche sichtbar bleibt.

9. Kombinationsarzneiform nach Anspruch 8, **dadurch gekennzeichnet, daß** es sich bei dem festen Präparat um eine erstarrte, wirkstoffhaltige Schmelze aus einem pharmakologisch akzeptablen polymeren Bindemittel (Bindemittel B), eine erstarrte Wirkstoffschmelze, eine Tablette, Pille, ein Pellet oder Wirkstoffkristalle handelt.

10. Kombinationsarzneiform nach Anspruch 9, wobei es sich bei dem Bindemittel B um eine Alkylcellulose, Hydroxyalkylcellulose, Carboxyalkylcellulose, Polyvinylpyrrolidon, ein Copolymerisat aus N-Vinylpyrrolidon und Vinylacetat, einen Zuk-keralkohol oder ein Polyethylenglykol handelt.

11. Kombinationsarzneiform nach einem der Ansprüche 8 bis 10 in Form einer Tablette.

## Claims

1. A process for producing solid combination drug forms which have at least two phases and are based on at least one pharmacologically acceptable polymeric binder and at least one pharmaceutical active ingredient, which comprises extruding a melt of the polymeric binder (binder A) with or without at least one active ingredient, and, during the extrusion, at least one solid product which may contain at least one active ingredient being taken up in the still plastic composition.

2. A process as claimed in claim 1, wherein the solid product used is a solidified melt of a pharmacologically acceptable polymeric binder (binder B) containing active ingredient, a solidified active ingredient melt, a tablet, pill, a pellet or active ingredient crystals.

3. A process as claimed in claim 2, wherein an alkylcellulose, hydroxyalkylcellulose, carboxyalkylcellulose, polyvinylpyrrolidone, a copolymer of N-vinylpyrrolidone and vinyl acetate, a sugar alcohol or a polyethylene glycol is used as binder B.

4. A process as claimed in claim 1, wherein the extrusion of the melt takes place by extruding and shaping in a calender with molding rolls which rotate in opposite directions and have on their surface depressions to receive and shape the melt.

5. A process as claimed in claim 4, wherein the solid product is introduced into the depressions of one molding roll.

6. A process as claimed in claim 5, wherein the melt of polymeric binder B containing active ingredient or the active ingredient melt is introduced into the depressions of one molding roll, and the melt is caused to solidify.

7. A process as claimed in any of claims 4 to 6, wherein a molding roll with depressions is combined with a smooth roll.

8. A solid combination drug form having at least two phases which has a first phase of at least one pharmacologically acceptable polymeric binder (binder A) in the form of a solidified melt which comprises, where appropriate, at least one pharmaceutical active ingredient and into which a solid product which contains at least one active ingredient is taken up as second phase, the second phase being incorporated in the first phase in such a way that part of its surface remains visible.

9. A combination drug form as claimed in claim 8, wherein the solid product is a solidified melt of a pharmacologically acceptable polymeric binder (binder B) containing active ingredient, a solidified active ingredient melt, a tablet, pill, a pellet or active ingredient crystals.

10. A combination drug form as claimed in claim 9, wherein the binder B is an alkylcellulose, hydroxyalkylcellulose, carboxyalkylcellulose, polyvinylpyrrolidone, a copolymer of N-vinylpyrrolidone and vinyl acetate, a sugar alcohol or a polyethylene glycol.

11. A combination drug form as claimed in any of claims 8 to 10 in the form of a tablet.

## Revendications

1. Procédé pour la préparation de formes médicamenteuses combinées solides, à au moins deux phases, à base d'au un liant polymère acceptable pour les usages pharmaceutiques et d'au moins une substance active pharmaceutique, **caractérisé par le fait que** l'on soumet une masse fondue du liant polymère (liant A) et le cas échéant au moins une substance active à formage, et lors du formage, on incorpore au moins une préparation solide, contenant le cas échéant au moins une substance active, dans la masse encore plastique.

2. Procédé selon la revendication 1, **caractérisé par le fait que** l'on utilise en tant que préparation solide, une masse fondue solidifiée, contenant une substance active, d'un liant polymère acceptable pour les usages pharmaceutiques (liant B), une masse fondue solidifiée contentant la substance active, un comprimé, une pilule, un granulé ou des cristaux de substance active.

3. Procédé selon la revendication 2, **caractérisé par le fait que** l'on utilise en tant que liant (B) une alkylcellulose, une hydroxyalkylcellulose, une carboxyalkylcellulose, une polyvinylpyrrolidone, un copolymère de N-vinylpyrrolidone et d'acétate de vinyle, un alcool de sucre ou un polyéthylèneglycol.

4. Procédé selon la revendication 1, **caractérisé par le fait que** le formage de la masse fondue est réalisé par extrusion et formage dans une calandre à cylindres de formage tournant en sens inverse, qui portent sur leur surface des cavités servant à absorber et à former la masse fondue.

5. Procédé selon la revendication 4, **caractérisé par le fait que** l'on incorpore la préparation solide dans les cavités de l'un des cylindres de formage.

6. Procédé selon la revendication 5, **caractérisé par le fait que** l'on incorpore la masse fondue, contenant une substance active, du liant polymère B ou la masse fondue contenant une substance active dans les cavités de l'un des cylindres de formage et on provoque la solidification de la masse fondue.

7. Procédé selon l'une des revendications 4 à 6, **caractérisé par le fait que** l'on combine un cylindre de formage portant des cavités et un cylindre lisseur.

8. Forme médicamenteuse combinée solide à au moins deux phases, dont une première phase comprenant au moins un liant polymère acceptable pour les usages pharmaceutiques (liant A) à l'état de masse fondue solidifiée, contentant le cas échéant au moins une substance active pharmaceutique, et dans laquelle on absorbe une deuxième phase consistant en une préparation solide qui contient au moins une substance active, la deuxième phase étant incorporée dans la première en sorte qu'une partie de sa surface reste visible.

9. Forme médicamenteuse combinée selon la revendication 8, **caractérisée par le fait que** la préparation solide consiste en une masse fondue solidifiée, contentant une substance active, d'un liant polymère acceptable pour les usages pharmaceutiques (liant B), une masse fondue solidifiée de substance active, un comprimé, une pilule, un granulé ou des cristaux de substance active.

10. Forme médicamenteuse combinée selon la revendication 9, dans laquelle le liant B consiste en une alkylcellulose, une hydroxyalkylcellulose, une carboxyalkylcellulose, une polyvinylpyrrolidone, un copolymère de N-vinylpyrrolidone et l'acétate de vinyle, un alcool de sucre ou un polyéthylèneglycol.

11. Forme médicamenteuse combinée selon l'une des revendications 8 à 10, à l'état de comprimé.
